# EUROPEAN PATENT APPLICATION

(11) **EP 1 520 592 A1**
(43) Date of publication of application: **06.04.2005**
(21) Application number: 03021992.7
(22) Date of filing: 30.09.2003
(51) Int. Cl.: A61L 11/00, B09B 3/00, B02C 19/12, A61L 2/06

(54) **Apparatus for the treatment of hospital waste**

(71) Applicant: Tecno Service First S.r.l., 47891 Dogana (SM)
(72) Inventor: Pericoli, Marco, 47900 Rimini (IT)
(74) Representative: Provvisionato, Paolo

(57) **Abstract**

An apparatus for the treatment of hospital waste, comprising a chamber (1) for containing the waste, a rotor (2) equipped with blades (3), situated inside the chamber (1) and able to crush the waste, a motor (4) situated outside the chamber (1) and able to actuate the rotor (2), and an air recirculating circuit (5) equipped with means (6) for heating the air output from the chamber (1) before reintroducing it into the said chamber (1). These air heating means (6) comprise at least one electrical resistor (6a) or, more generally, a set of electrical resistors (6a, 6b, 6c, etc.). A device (7) for controlling the temperature of the chamber (1) converts the variations in resistance into variations in temperature so that the evolution of the latter within the industrial plant may be monitored.

## Description

The present invention concerns an apparatus for the treatment of hospital waste.

In hospitals and sanitary structures in general it is of primary importance to solve the problem of waste treatment, for which very high temperatures are often required in order to ensure sterilization and disinfection.

There are obviously many ways of meeting this requirement: these include methods in which transmission of the heat is performed before the waste is crushed, by blowing in saturated steam, in the case of wet processes, or hot air or superheated steam, in the case of dry processes; in other methods the waste containers are heated externally or microwave irradiation is used, although the latter method is often unable to achieve high temperatures owing to the need for water on which the microwaves must act. Moreover, a more recent method, forming the subject of Italian patent No. 1,260,938, is able to achieve the desired temperatures (150°-200°), by producing energy from the impacts and the friction produced in the waste by means of a rotor provided with blades during the operations involving crushing and pulverization of the said waste.

While the first methods require cycles of long duration or are unable to reach the necessary temperatures, the latter method has an operational limitation in that the density of the product to be disposed of might not be able to offer adequate resistance to the blades of the rotor and therefore fail to generate, by means of friction, a sufficient amount of energy for reaching the desired temperatures.

The object of the present invention is therefore that of eliminating the abovementioned drawbacks.

This is achieved by means of an air recirculating circuit which removes air from the chamber containing the waste and reintroduces it therein after suitably raising the temperature thereof.

The main advantage obtained by means of the present invention consists essentially in the fact that the apparatus thus equipped is highly flexible so that it can be adapted to any quantity of waste to be treated, whether it be hospital waste or waste from other sources.

Further advantages and characteristics of the invention will emerge more clearly from the detailed description which follows, provided with reference to the accompanying drawings which illustrates a non-limiting example of embodiment thereof, where:

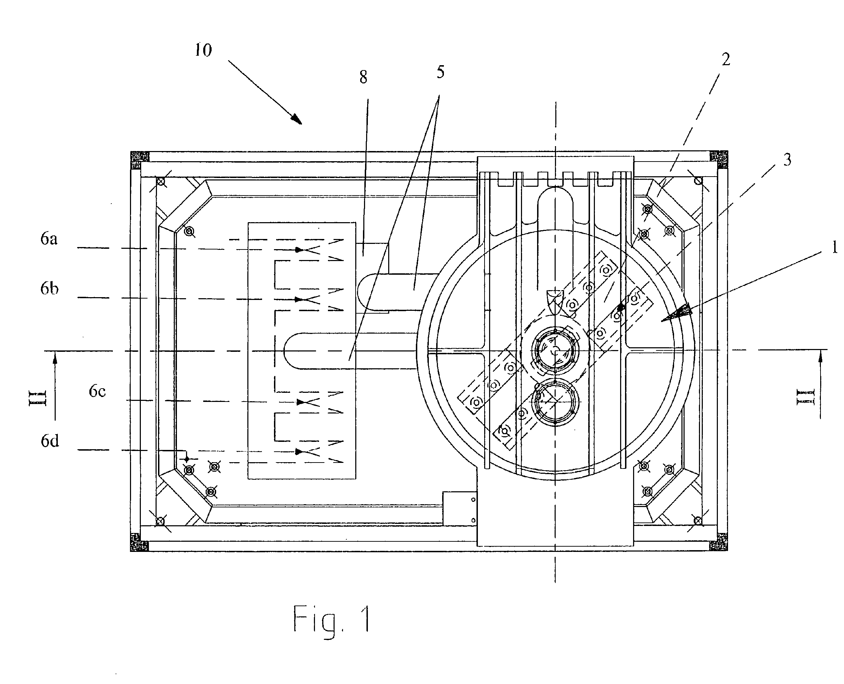

- Fig. 1 shows a top view in plant of the invention, with some parts removed for better emphatizing some others;

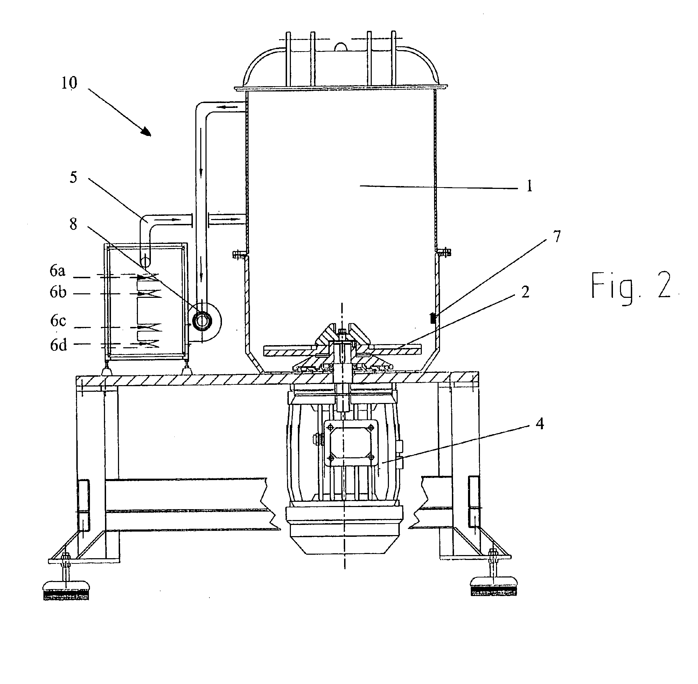

- Fig. 2 shows a cross-sectional view of the invention along II-II in Figure 1;

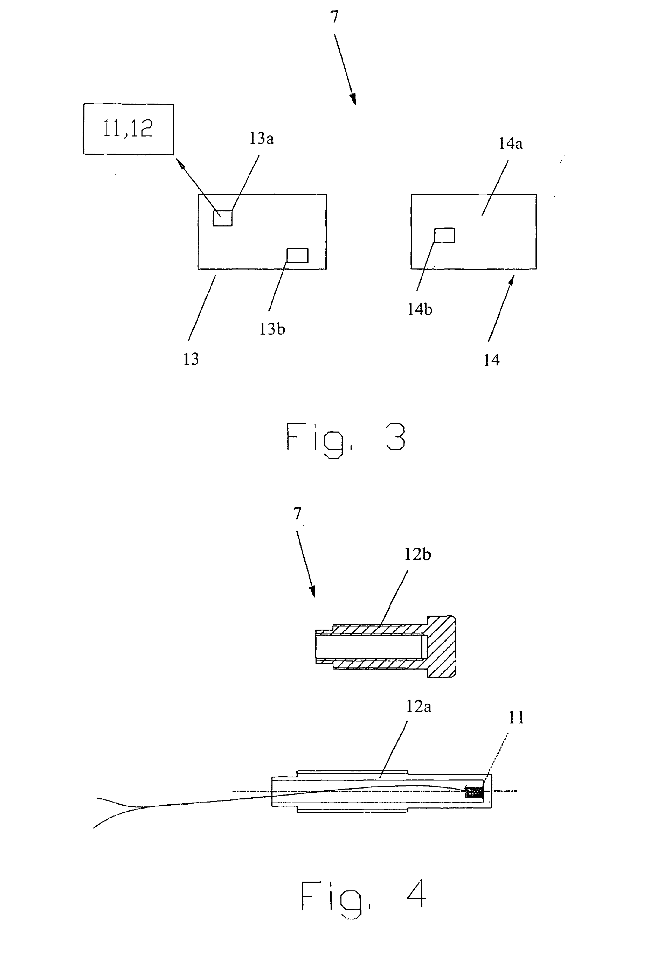

- Fig. 3 and 4 show a detail of the invention.

As can be seen from the figures, the invention concerns an apparatus for the treatment of hospital waste comprising a chamber (1) for containing the waste, a rotor (2) equipped with blades (3), situated inside the chamber (1) and able to crush the waste, a motor (4) situated outside the chamber (1) and able to actuate the rotor (2), and an air recirculating circuit (5) equipped with means (6) for heating the air output from the chamber (1) before reintroducing it into the said chamber (1) .

The air heating means (6) comprise at least one electrical resistor (6a), more precisely, a set of electrical resistors (6a, 6b, 6c, ..), the number of which varies depending on the size of the plant, which is defined on the basis of quantity of infected material to be sterilized for each working hour of the plant.

A fan (8) extracts a fixed quantity of air from the chamber (1) turning it toward the electrical resistors (6a, 6b, 6c...) before its reintroduction in the chamber itself.

The apparatus also comprises a device (7) for controlling the temperature of the chamber (1). Said device is formed in turn by a resistance sensor (11) which can be stably associated with a high-conductivity protective cover (12), an interface (13) able to convert the variations in resistance into variations in current or voltage, and means (14) for converting the current or voltage variations into variations in temperature so that the evolution thereof within the plant may be monitored.

The protective cover (12) comprises two portions (12a, 12b) which can be separated from each other, i.e. a first internal portion (12a) which is in direct contact with the resistance sensor (11) and a second external portion (12b) which is directly exposed inside the plant so that the deterioration due to use is concentrated on the second portion (12b) of the protective cover (12).

The interface (13) comprises at least one electronic component (13a) able to produce a current of constant intensity to be sent to the resistance sensor (11) and, preferably, at least one second electronic component (13b) for stabilizing the output current.

The means (14) for converting the current or voltage variations into temperature variations comprise at least one data processor (14a) provided with a data bank (14b) which establishes a correspondence between the variation in voltage or current supplied by the interface (13) and the variation in temperature detected by the resistance sensor (11).

The chamber (1) containing the waste has been suitably designed with variable dimensions so as to be adaptable to different quantities of waste and, correspondingly, different quantities of air to be circulated within the circuit (5).

Purely by way of example, and without any type of limitation of the scope arising from the present patent application, it may be useful to reproduce the data relating to a practical example of use of the apparatus (10) in question.

From experience and according to international literature it is possible to state that the percentage moisture content of potentially infected sanitary waste is equivalent to about 20%, so that it may be considered that 200 grammes of water and 800 grammes of dry fraction are present for each kilo of material. On this basis the energy necessary for heating one kilo of material from 20°C to 150°C may be calculated as follows: 16,000 calories for heating 200 grammes of water from 20°C to 100°C; 120,000 calories for obtaining evaporation of the said 200 grammes of water; 31,200 calories for heating the dry fraction from 20°C to 150°C (assuming for the latter a specific heat of 0.3 calories per degree centigrade). This gives a total of 167.2 kilocalories.

Considering a fairly significant loss of heat, in the region of 50%, i.e. a thermal efficiency of the plant equal to about 0.5, the abovementioned energy requirement must be doubled and, therefore, for each kilo of material about 325 kilocalories will be necessary.

Converting this value firstly into Joules and then into watt-hours, it is established that about 0.4 kWh are required to sterilize one kilo of material.

Therefore, equipping the apparatus (10) with an air recirculating circuit comprising a set of four electrical resistors (6a, 6b, 6c, 6d) of 2.5 kW each, with an overall power of 10 kW, there is the possibility of disposing of 25 kilos of hospital waste for each working hour.

Obviously, as moreover already occurs in numerous plants of this type, it is also possible to perform the addition of plastic materials and/or antioxidants so as to improve the sterilization efficiency. The power of the air heating means (6) will be recalculated in each case on the basis of the composition of the mixture present in the chamber (1).

The invention thus devised may be subject to numerous modifications and variants, all falling within the scope of the inventive idea. Moreover, all the details may be replaced by technically equivalent elements.

## Claims

1. Apparatus for the treatment of hospital waste, comprising a chamber (1) for containing the waste, a rotor (2) equipped with blades (3), situated inside the chamber (1) and able to crush the waste, and a motor (4) situated outside the chamber (1) and able to actuate the rotor (2), **characterized in that** it comprises an air recirculating circuit (5) equipped with means (6) for heating the air output from the chamber (1) before reintroducing said air into the chamber (1).

2. Apparatus according to Claim 1, **characterized in that** the air heating means (6) comprise at least one electrical resistor (6a).

3. Apparatus according to Claim 1, **characterized in that** the air heating means (6) comprise a set of electrical resistors (6a, 6b, 6c, etc.)

4. Apparatus according to Claim 1, **characterized in that** the chamber (1) has a variable volume so as to be adaptable to the quantity of waste to be treated.

5. Apparatus according to Claim 1, **characterized in that** it comprises a fan (8) able to extract air from the chamber (1) and to turn it toward the means (6) to warm the air before its reintroduction in the chamber (1) itself.

6. Apparatus according to Claim 1, **characterized in that** it comprises a device (7) for controlling the temperature of the chamber (1).

7. Apparatus according to Claim 6, **characterized in that** said device (7) comprises a resistance sensor (11) which can be stably associated with a high-conductivity protective cover (12), an interface (13) able to convert the variations in resistance into variations in current or voltage, and means (14) for converting the current or voltage variations into variations in temperature so that the evolution thereof within the industrial plant can be monitored.

8. Apparatus according to Claim 7, **characterized in that** the protective cover (12) comprises two portions (12a, 12b) which can be separated from each other, i.e. a first internal portion (12a) which is in direct contact with the resistance sensor (11) and a second external portion (12b) which is directly exposed inside the plant so as to concentrate the deterioration due to use on the second portion (12b) of the protective cover (12).

9. Apparatus according to Claim 75, **characterized in that** the interface (13) comprises at least one electronic component (13a) able to produce a current of constant intensity to be sent to the resistance sensor (11).

10. Apparatus according to Claim 7, **characterized in that** the interface (13) comprises at least one second electronic component (13b) for stabilizing the output current.

11. Apparatus according to Claim 7, **characterized in that** the means (14) for converting the current or voltage variations into temperature variations comprise at least one data processor (14a) provided with a data bank (14b) which establishes a correspondence between the variation in voltage or current supplied by the interface (13) and the variation in temperature detected by the resistance sensor (11).
